# EUROPEAN PATENT APPLICATION

(11) **EP 4 696 243 A1**
(43) Date of publication of application: **18.02.2026**
(21) Application number: 25190667.3
(22) Date of filing: 21.07.2025
(51) Int. Cl.: A61B 6/58, A61B 6/03, A61B 6/00

(54) **METHODS AND SYSTEMS FOR INTEGRATED PHANTOM SYSTEM**

(30) Priority: 15.08.2024 US 202418806614
(71) Applicant: GE Precision Healthcare LLC, Waukesha, WI 53188 (US)
(72) Inventor: DELONG SAMALIK, Michelle Marie Severino, Waukesha, 53188-1696 (US); LEWIS, Chelsey Amanda, Waukesha, 53188-1696 (US); SMITH, Brandon A., Waukesha, 53188-1696 (US)
(74) Representative: AWA Sweden AB

(57) **Abstract**

Various methods and systems are provided for a slab phantom set of an imaging system. The imaging system (200) comprises a gantry (322) including a radiation source (302) and a detector (314). A housing (306) is further positioned in the gantry, adjacent to the radiation source. The housing includes a slab phantom set (304) that comprises at least one slab phantom (324, 326). The slab phantom set is configured such that at least one slab phantom of the slab phantom set is independently movable to enable one or more of the at least one slab phantom of the slab phantom set is to be selectively positioned in a path (312) of a radiation beam (308) between the radiation source and the detector.

## Description

### FIELD

Embodiments of the subject matter disclosed herein relate to diagnostic medical imaging, and more particularly, to computed tomography imaging setup with an integrated phantom assembly.

### BACKGROUND

In computed tomography (CT imaging systems, an electron beam generated by a cathode is directed towards a target within an X-ray tube. A fan-shaped or cone-shaped beam of X-rays produced by electrons colliding with the target is directed toward a subject, such as a patient. After being attenuated by the object, the X-rays impinge upon an array of radiation detector elements. An electrical signal is generated at each detector element, and the electrical signals generated at the detector elements are used to reconstruct an image of the object, where each electrical signal corresponds to a voxel/pixel of the image.

A quality of the image in terms of resolution, contrast-to-noise ratio, and other factors may depend on an alignment of each detector element within the detector arrays. Misalignments of the detector elements may increase a number of artifacts in the image and/or reduce the quality of the image. A calibration process may be performed periodically on the system to obtain projection data of materials that simulate varying human tissue densities. The calibration process may include performing an X-ray scanning procedure on an object, called a phantom. Physical misalignment of a phantom during a calibration process may result in inaccurate calibration of the CT system.

### BRIEF DESCRIPTION

Various methods and systems are provided for a slab phantom set of an imaging system. The imaging system comprises a gantry including a radiation source and a detector. A housing is further positioned in the gantry, adjacent to the radiation source. The housing includes a slab phantom set that comprises at least one slab phantom. The slab phantom set is configured such that the at least one slab phantom of the slab phantom set is independently movable to enable one or more of the at least one slab phantom of the slab phantom set to be selectively positioned in a path of a radiation beam between the radiation source and the detector.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 shows a pictorial view of an imaging system according to an embodiment of the invention.
FIG. 2 shows a block schematic diagram of the imaging system including a gantry, where the gantry houses a radiation source, a detector, a beam-filtering device, and a housing positioned adjacent to the radiation source.
FIG. 3 shows a configuration of the imaging system of FIG. 2, where elements of the beam-filtering device and a slab phantom set are housed in the housing.
FIG. 4 shows a configuration of the imaging system of FIG. 2, where elements of the beam-filtering device are housed in the beam-filtering device and where the slab phantom set is positioned in the housing.
FIG. 5 shows the configuration of the imaging system of FIG. 4, where slab phantoms of the slab phantom set are positioned in a path of an X-ray beam emitted by the radiation source.
FIG. 6 shows a side view of the configuration of the imaging system of FIG. 2.
FIGS. 7A-7B show side views of the configuration of the imaging system of FIG. 2 with various bowtie filters of the beam-filtering device and/or slab phantoms of the slab phantom set positioned in the path of the X-ray beam.
FIGS. 8A-8B show a configuration of the imaging system of FIG. 2, where the beam-filtering device and the housing include slab phantoms of the slab phantom set.
FIG. 9 shows a configuration of the imaging system of FIG. 2 where one or more slab phantoms of the slab phantom set may be moveable by a single motor.
FIG. 10 shows side views of a configuration of the imaging system of FIG. 2, where the housing is configured to be selectively positioned in the path of the X-ray beam via a hinge.
FIG. 11 shows side views of the configuration of FIG. 10.
FIG. 12 shows a configuration of the imaging system of FIG. 2, where the housing is configured to be selectively positioned in the path of the X-ray beam via a linear track.
FIG. 13 shows a flow chart for a method for performing calibration procedures of the imaging system.

### DETAILED DESCRIPTION

The following description relates to various embodiments of an X-ray imaging system. Some imaging systems, such as computed tomography (CT) systems or photon counting computed tomography (PCCT) systems may demand relatively regular calibration, such as daily or weekly calibration scans to offset any gain drive, for example realized from hardware such as X-ray tube focal spot position changes or radiation degradation of the detectors. Further, PCCT or CT systems may obtain spectral information that allows generation of basis material decomposition (BMD) images. Calibrating PCCT systems may thereby demand that calibration projection data be obtained that mimics the materials and material thicknesses of the human body. Thus, phantoms for calibrating PCCT systems may include multiple different materials, such as polyvinyl chloride (PVC) and polyethylene (PE). Phantoms of multiple types are available for calibration purposes, including slab phantoms, step phantoms, pillar phantoms, and more.

Calibration using slab phantoms often demand selection of different slabs of different thicknesses, densities, and/or materials to form a single phantom to be used in a calibration scan protocol. These slabs are placed in the bore, usually attached to the table via an accessory slot at the front of the table. The process of forming such slab phantoms and positioning them within the imaging system is time-consuming and prone to human error (e.g., in positioning, slab selection, and the like). Further, slab phantoms that include slabs of multiple types of materials and densities are often large, heavy, and physically cumbersome to move around. Additionally, the large slabs take up storage space in facilities that may otherwise be demanded for storage of other materials or supplies.

Thus, systems and methods are herein provided that at least partially address these issues. In particular, systems and methods for an integrated phantom system are herein provided. The integrated phantom system as herein disclosed includes slab phantoms stored within a gantry of the imaging system. For example, a slab phantom set may be stored within a beam-filtering device (e.g., a housing of a collimator that also stores filters such as bowtie filters that are used in diagnostic protocols) or within a separate housing adjacent to the radiation source. Each slab phantom of the set of slab phantoms may be individually actuated to move into a field of view of the X-ray source (e.g., a path from the radiation source to the detector) based on a selected calibration protocol, thus reducing time spent by an operator in manually selecting and assembling a phantom with multiple slabs. Further, as the housing in which the slab phantoms are position is adjacent to the radiation source of the imaging system, a field of view that is intersected by the phantom may be reduced, which in turn allows for reduced size and overall footprint of the phantom slabs. The slab phantoms may be automatically moved into and out of the X-ray beam, which may allow for faster and more accurate placement of one or more slab phantoms, for more efficient calibration processes.

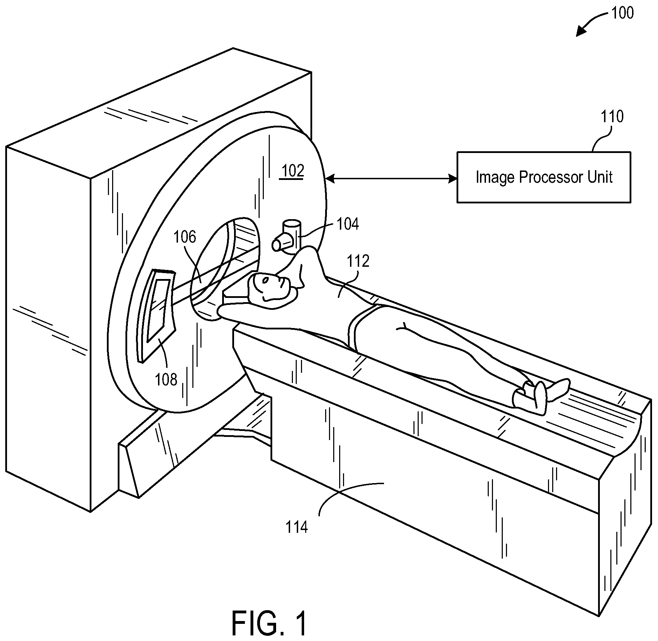

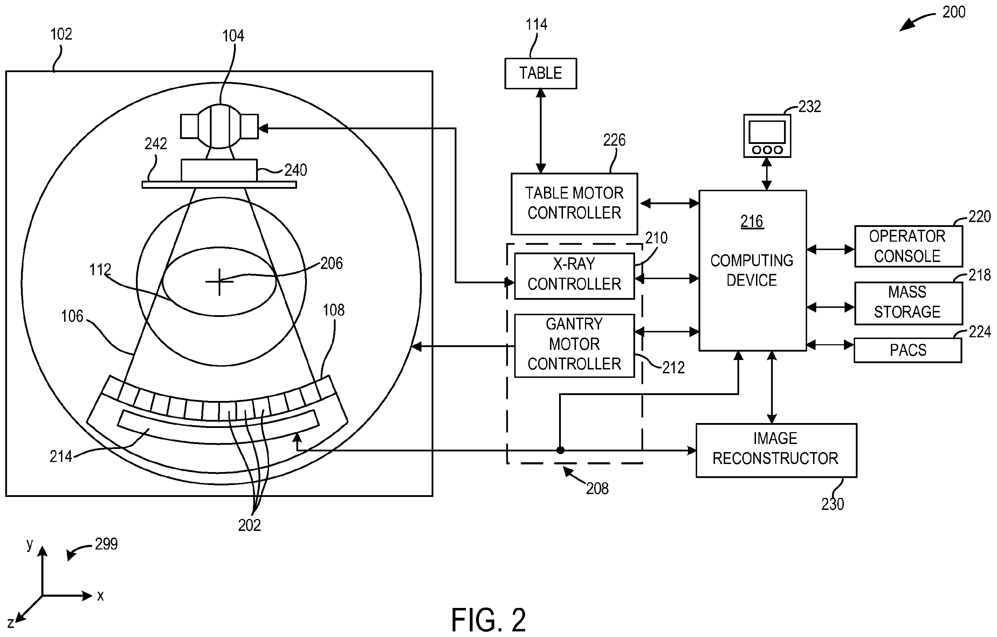

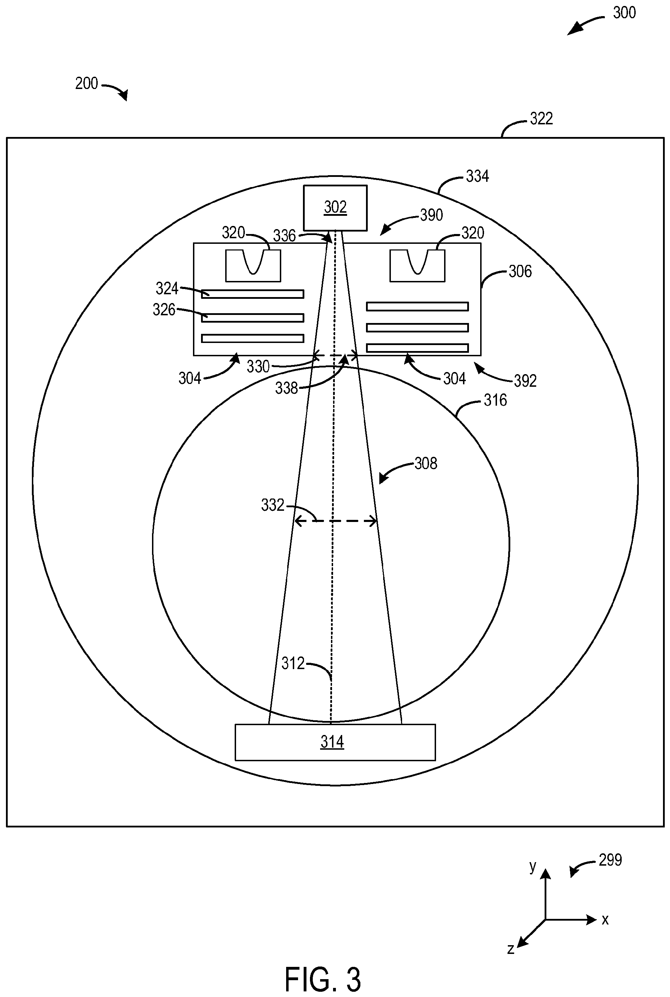

The systems and methods herein disclosed will now be described, by way of example, with respect to the figures, wherein FIGS. 1 and 2 show an exemplary imaging system, FIGS. 3-12 show various configurations of a slab phantom system integrated into the imaging system of FIGS. 1-2, and FIG. 13 shows a flowchart illustrating a method for slab selection in an integrated phantom system.

FIG. 1 illustrates an exemplary CT system 100. In one example, the CT system 100 may be a PCCT system. Particularly, the CT system 100 is configured to image a subject 112 such as a patient, an inanimate object, one or more manufactured parts, and/or foreign objects such as dental implants, stents, and/or contrast agents present within the body or subject placed on a table 114. The table 114 may be motorized and may be selectively moveable. In one embodiment, the CT system 100 includes a gantry 102, which in turn, may further include at least one X-ray radiation source 104 configured to project an X-ray beam 106 for use in imaging the subject 112. The X-ray radiation source 104 includes an X-ray tube and a target. The X-ray tube generates X-rays by accelerating and focusing a high-energy beam of electrons onto a rotating target. As individual electrons strike the target, the energy released by interacting with the atoms of the target produces X-ray photons isotropically under a polychromatic spectrum, a maximum energy of the X-ray photons matching that of the incident electrons. The X-ray photons leave the tube through a window that defines an X-ray beam. The beam can then be collimated and conditioned using collimator blades and filter(s).

Specifically, the radiation source 104 is configured to project the X-ray beam 106 towards a detector array 108 positioned on the opposite side of the gantry 102. Although FIG. 1 depicts a single radiation source 104, in certain embodiments, multiple radiation sources may be employed to project a plurality of X-ray beams 106 for acquiring projection data corresponding to the subject 112 at different energy levels. The radiation source may include an X-ray target manufactured of graphite and metal.

In certain embodiments, the CT system 100 further includes an image processing unit 110 configured to reconstruct images of a target volume of the subject 112 using an iterative or analytic image reconstruction method. For example, the image processing unit 110 may use an analytic image reconstruction approach such as filtered backprojection (FBP) to reconstruct images of a target volume of the subject 112. As another example, the image processing unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112.

FIG. 2 illustrates an exemplary imaging system 200 similar to the CT system 100 of FIG. 1. The imaging system 200 includes at least some of the elements of the CT system 100. In one embodiment, the system 200 includes the detector array 108. The detector array 108 further includes a plurality of detector elements 202 that together sense the X-ray beam 106 that pass through the subject 112 such as a patient to acquire corresponding projection data. Accordingly, in one embodiment, the detector array 108 is fabricated in a multi-slice configuration including the plurality of rows of cells or detector elements 202. In such a configuration, one or more additional rows of the detector elements 202 are arranged in a parallel configuration for acquiring the projection data.

A beam-filtering device 240 may be mounted within the gantry 102 between the radiation source 104 and the subject 112. The beam-filtering device 240 may have components that travel in and out of the beam in the z-direction while the beam is substantially in the y-direction. The beam-filtering device 240 may be configured to store one or more filters, such as bowtie filters configured for use in diagnostic protocols, as well as one or more slab phantoms, configured for use in calibration protocols, in some examples. The beam-filtering device 240 may perform the functions of a pre-patient collimator (filtration and collimation of the beam for diagnostic imaging) and/or filtering of the beam for other purposes such as calibrations. In some examples, a housing 242 may be positioned adjacent to and separate from the beam-filtering device 240, wherein the beam-filtering device 240 stores one or more filters, such as bowtie filters, and the housing 242 stores one or more phantom slabs. The housing 242 for a slab phantom set as described herein with respect to FIG. 3 may be positioned directly adjacent to the beam-filtering device 240 within the rotating portion of the gantry 102 of the imaging system 200. In either example, phantom slabs may be moved into and out of the beam depending on a selected calibration protocol separate from the one or more filters, which may be moved into and out of the beam based on a selected diagnostic protocol.

In certain embodiments, the system 200 is configured to traverse different angular positions around the subject 112 for acquiring desired projection data. Accordingly, the gantry 102 and the components mounted thereon (such as the radiation source 104, the beam-filtering device 240, the housing 242, and the detector array 108) may be configured to rotate about a center of rotation 206 for acquiring the projection data, for example, at different energy levels. Alternatively, in embodiments where a projection angle relative to the subject 112 varies as a function of time, the mounted components may be configured to move along a general curve rather than along a segment of a circle.

In one embodiment, the system 200 includes a control mechanism 208 to control movement of the components such as rotation of the gantry 102 and the operation of the X-ray radiation source 104. In certain embodiments, the control mechanism 208 further includes an X-ray controller 210 configured to provide power and timing signals to the radiation source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The data sampled and digitized by the DAS 214 is transmitted to a computing device (also referred to as processor) 216. In one example, the computing device 216 stores the data in a storage device 218. The storage device 218, for example, may include a hard disk drive, a floppy disk drive, a compact disk-read/write (CD-R/W) drive, a Digital Versatile Disc (DVD) drive, a flash drive, and/or a solid-state storage device.

Additionally, the computing device 216 provides commands and parameters to one or more of the DAS 214, the X-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard or a touchscreen to allow the operator to specify the commands and/or scanning parameters.

Although FIG. 2 illustrates one operator console 220, more than one operator console may be coupled to the system 200, for example, for inputting or outputting system parameters, requesting examinations, and/or viewing images. Further, in certain embodiments, the system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks.

In one embodiment, for example, the system 200 either includes, or is coupled to a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control the table 114. For example, the table 114 may be motorized via a motor. The table motor controller 226 may actuate the motor of the table 114 to move the table 114 for appropriately positioning the subject 112 in the gantry 102 for acquiring projection data corresponding to the target volume of the subject 112.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the system 200 and instead the computing device 216 may perform one or more functions of the image reconstructor 230. Moreover, the image reconstructor 230 may be located locally or remotely, and the image reconstructor 230 may be operatively connected to the system 100 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 transmits the reconstructed images to the computing device 216 for generating useful patient information for diagnosis and evaluation. In certain embodiments, the computing device 216 transmits the reconstructed images and/or the patient information to a display 232 communicatively coupled to the computing device 216 and/or the image reconstructor 230.

As further described herein with respect to FIGS. 3-13, the imaging system 200 may be calibrated by executing one or more of a calibration scan protocol. A user may request the calibration scan protocol via the operator console 220. The computing device 216 may identify a calibration scan protocol requested, and determine a slab phantom corresponding to the identified calibration scan protocol. The slab phantom may include one or more slab phantoms of the slab phantom set that are housed in the beam-filtering device 240 and/or the housing 242. The computing device 216 may identify a subset of slabs of the slab phantom set incorporated into the housing 242 and/or the beam-filtering device 240 of the imaging system 200 that form the slab phantom corresponding to the identified calibration scan protocol. The computing device 216 may actuate one or more motors that are coupled to the identified subset of slabs to move the subset of slabs into a path of a radiation beam (e.g., the X-ray beam 106). The imaging system 200 may thus acquire a calibration scan of the slab phantom.

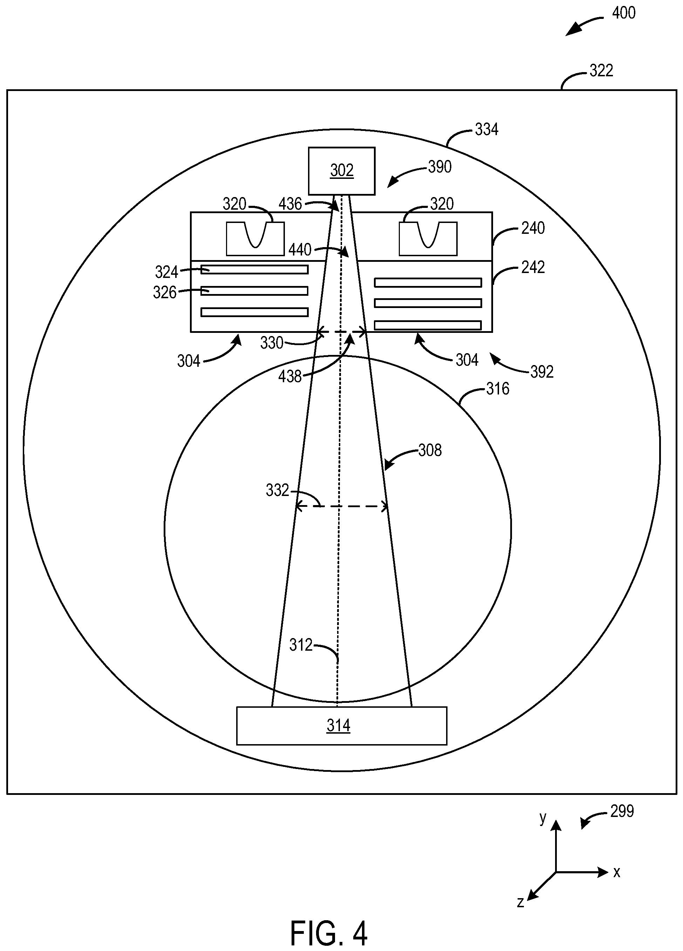

FIG. 3 shows a configuration 300 of the imaging system 200 of FIG. 2, where the gantry 102 houses the radiation source 104, the beam-filtering device 240, the housing 242, and the detector array 108. In the configuration shown in FIG. 3, the housing 242 and the beam-filtering device 240 are combined as a single housing 306 that includes elements of the beam-filtering device 240 and elements of the housing 242. For example, a slab phantom set 304 is positioned in the single housing 306 of the imaging system 200, as further described herein. Additionally, in this illustrated example, one or more example bowtie filters 320 are positioned in the single housing 306. The one or more example bowtie filters 320 may alternatively be positioned in the beam-filtering device 240, as further described with respect to FIGS. 4-12. In this illustrated example, phantoms of the slab phantom set 304 and other filters of the imaging system 200 (e.g., bowtie filters) are moving parallel to the x-axis. Alternatively, the slab phantom set 304 and the single housing 306 may be positioned such that the slab phantom set 304 moves parallel to the z-axis. FIG. 3 includes an axis system 299, wherein an x-axis is a lateral axis, a y-axis is a vertical (e.g., gravitational) axis, and a z-axis is a longitudinal axis (e.g., in and out of the gantry).

The configuration 300 of the imaging system 200 comprises an X-ray source 302 (e.g., the radiation source 104 of FIGS. 1-2) that is configured to emit an X-ray beam (e.g., X-ray beam 106) 308. The X-ray beam 308 travels along a path 312 from the X-ray source 302 to a detector 314 (e.g., the detector array 108) of the imaging system 200. The detector 314 is positioned within a rotating portion 334 of a gantry housing 322 (e.g., the gantry housing 102 of FIGS. 1 and 2). The detector 314 is positioned on an opposite side of a bore 316 of the gantry housing 322 from the X-ray source 302. The rotating portion 334 further houses the X-ray source 302 and the single housing 306 that contains the slab phantom set 304. The single housing 306 is positioned within the rotating portion 334 adjacent to the X-ray source 302 such that the single housing 306 is in the path 312 of the X-ray beam 308.

In the example of FIG. 3, the single housing 306 is made of metal (e.g., aluminum). Alternatively, the housing may be made of an X-ray transparent material. The single housing 306 includes a first gap 336 on a first side 390 of the single housing 306 adjacent to the X-ray source 302 and a second gap 338 on a second side 392 of the single housing 306 opposite to the first side 390. The first and second gaps 336, 338 are aligned (e.g., along the y-axis) such that the X-ray beam 308 passes through the first and second gaps 336, 338. The single housing 306 includes a collimator that is configured to align and/or narrow the X-ray beam 308. For example, the single housing 306 may include one or more bowtie filters 320. When positioned in the path 312 of the X-ray beam 308, the one or more bowtie filters 320 are configured to alter one or more configurations of the X-ray beam, for example to reduce an amount of radiation delivered to peripheries of a patient. In some examples, one bowtie filter 320 is positioned in the path of the X-ray beam 308. In some examples, more than one filter (e.g., another bowtie filter 320, a different filter) may be positioned in the path of the X-ray beam 308 with one of the example bowtie filters 320. The bowtie filter(s) 320 is positioned in the single housing 306 at the first side 390 of the single housing 306. The bowtie filter(s) 320 may be configured for use in diagnostic imaging protocols, and may be moved into and out of intersection with the X-ray beam 308 during diagnostic protocols. In contrast, the slab phantom set 304 may be configured for use in calibration protocols, and may be moved into and out of intersection with the X-ray beam 308 during calibration scans.

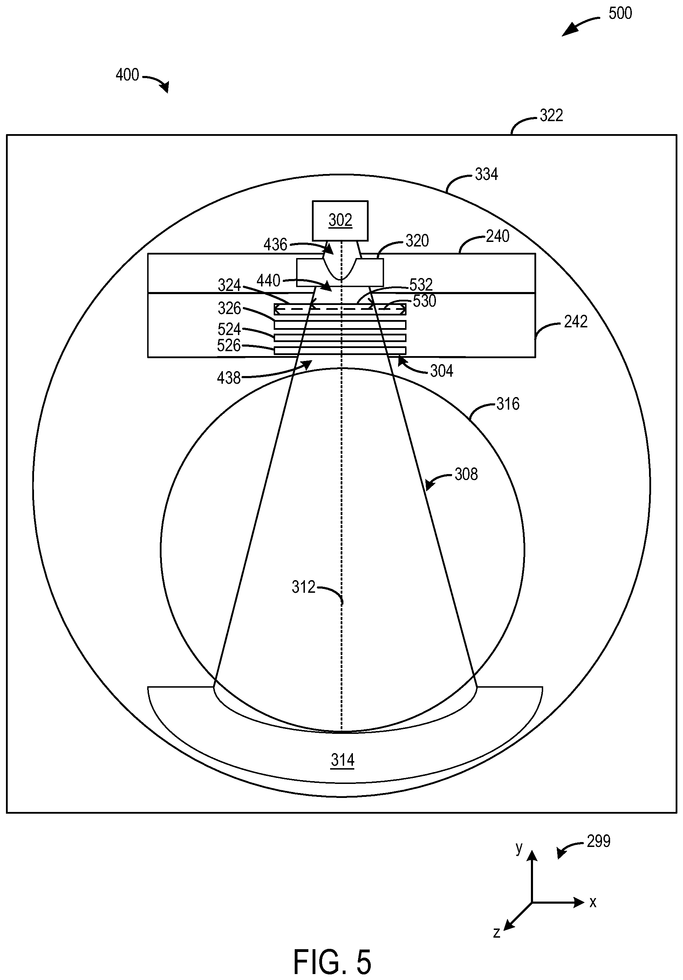

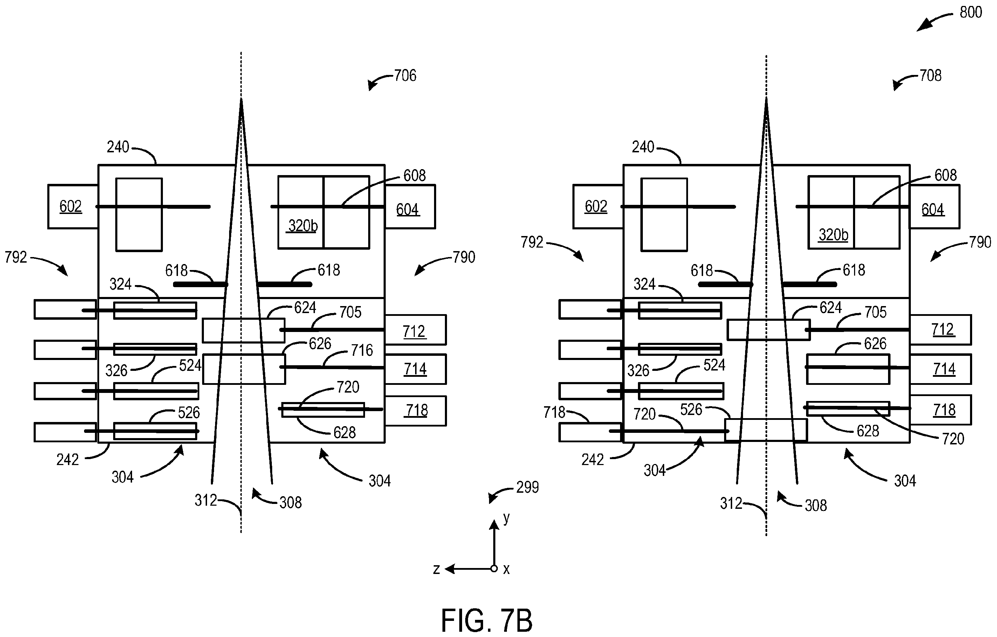

In the embodiment shown in FIG. 3, the slab phantom set 304 is positioned in the single housing 306. The slab phantom set 304 comprises multiple phantom slabs. As described herein, a slab phantom is a piece of material configured to mimic a particular density of human tissue (e.g., bone, blood, brain matter, etc.). With known parameters of the slab, and a group of slabs that form a chosen slab phantom, calibration of the imaging system 200 may be performed by comparing various outputs of the calibration scan to a standard. The slab phantom set 304 may comprise a first slab phantom 324 having a first thickness, where the first thickness is parallel to the direction of the path 312 (e.g., parallel to the y-axis). The first slab phantom 324 is formed of a first material. The slab phantom set 304 may further include a second slab phantom 326 having a second thickness, where the second thickness is parallel to the direction of the path 312 (e.g., parallel to the y-axis). The second slab phantom 326 is formed of a second material. The second thickness may be different than the first thickness. The second material may be different than the first material. The slab phantom set 304 may include additional slab, each of which may be formed of the same and/or a different material than the first material and/or the second material. In the example of FIG. 3, the slab phantom set 304 comprises six phantom slabs. Different combinations of the six available slabs may be pre-programmed as slab phantoms in the system, whereby selection of a first slab phantom may trigger selection of a first subset of the available slabs. The additional slabs may each have a thickness, parallel to the y-axis, that is greater than, less than, and/or equal to the first thickness and/or the second thickness. Each of the additional slabs may have a different thickness. Spaces between the slabs of the slab phantom set 304 (e.g., between the first slab phantom 324 and the second slab phantom 326 along the y-axis) may enable multiple slabs to be stacked in the path 312 of the X-ray beam 308, along the y-axis, to create a single filtering phantom. Example combinations of slab phantoms are described with respect to FIGS. 5 and 7B.

The slab phantom set 304 is positioned at the second side 392 of the single housing 306 opposite the first side 390. The single housing 306 is oriented such that the bowtie filter(s) 320 is close to the X-ray source 302 (e.g., with no components of the single housing 306 between the bowtie filter(s) 320 and the X-ray source 302). Described another way, the slab phantom set 304 and the bowtie filter(s) 320 may be vertically stacked with respect to the y-axis. The slab phantom set 304 may be positioned away from the X-ray source 302 with the bowtie filter(s) 320 and a wall of the single housing 306 at the first side 390 of the single housing 306 therebetween. Thus, the slab phantom set 304 may be incorporated into the same housing as the bowtie filters, allowing for storage of the slabs within the imaging system rather than external to the imaging system.

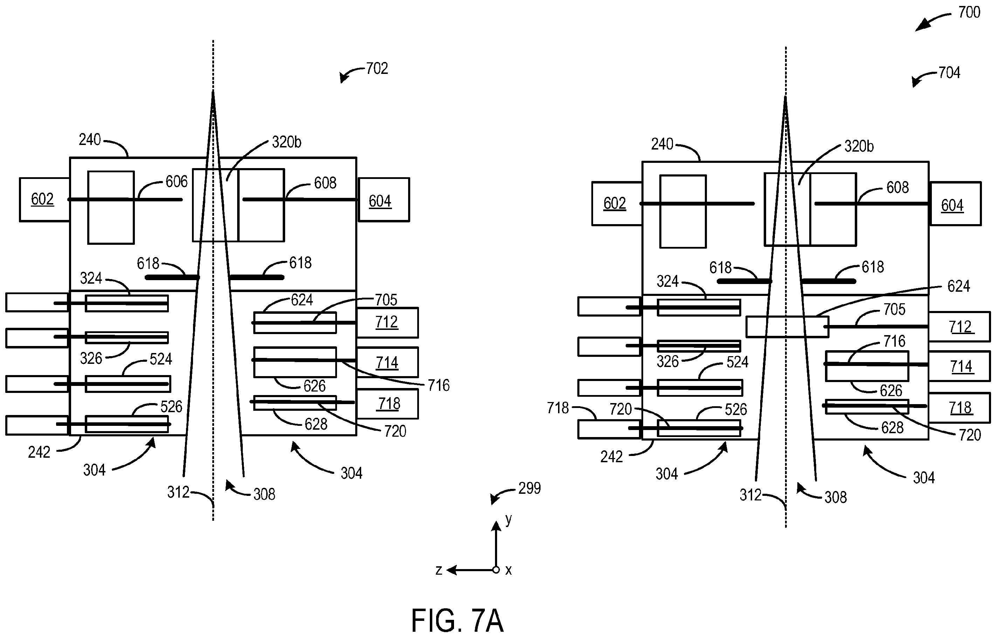

Additionally, by positioning the slab phantom set 304 closer to the X-ray source 302 than to the detector 314 (e.g., on a same side of the bore 316 as the X-ray source 302, opposite the detector), a size of each slab of the slab phantom set 304 may be decreased compared to conventional slab phantoms. For example, in conventional calibration procedures for calibrating an imaging system (e.g., the imaging system 200), one or more slab phantoms may be positioned in the path 312 of the X-ray beam 308 by placing the one or more slabs on an imaging surface (e.g., the table 114). The imaging surface is inserted into the bore 316. As is shown in FIG. 3, a width of the X-ray beam 308 in the bore 316 is greater than a width of the X-ray beam 308 outside of the bore 316, closer to the X-ray source 302. For example, a first width 332 of the X-ray beam 308 in the bore 316 is greater than a second width 330 of the X-ray beam 308 that passes through the single housing 306. In order to image an entirety of the selected slab phantom (e.g., such that the X-ray beam 308 received by the detector 314 passes through the slab phantom), slabs that are moved to intersect with the X-ray beam 308 are configured to have a width (e.g., parallel to the x-axis) that is at least equal to, and/or greater than, the width of the X-ray beam 308 at the position where the phantom is positioned in the X-ray beam 308. Therefore, by positioning the slab phantom set 304 and slabs thereof closer to the X-ray source 302, a width of each of the slabs may be smaller than a width of a slab phantom positioned further along the path 312 of the X-ray beam 308 (e.g., closer to the detector 314), while achieving the same desired filtering. A footprint and a weight of each slab phantom, and of the slab phantom set 304, may thus be decreased. Further, as the slab phantom set 304 is stored in the single housing 306 that is integrated in the imaging system 200, a complexity, space, and work demand for storing the slab phantom set 304 may be reduced and/or eliminated. Additionally, as described with respect to FIGS. 7-13, one or more slab phantoms of the slab phantom set 304 may be automatically moved into and/or out of the X-ray beam 308 during different calibration procedures of the imaging system 200. This further reduces a demand on a user to manually move and/or adjust positioning of one or more slab phantoms in the path 312 of the X-ray beam 308 (e.g., on the table 114.) Thus, a time taken to execute the calibration procedure may be decreased. Additionally, an accuracy of the imaging system calibration may be increased by automating positioning of slab phantoms in the X-ray beam 308 in response to selection of one or more calibration procedures.

The geometric configuration of the single housing 306 that includes the slab phantom set 304 and one or more bowtie filters 320 shown in FIG. 3 may be implemented in a CT imaging system having a generally smaller detector 314 span in the x-direction. In this shown configuration the bowties 320 and slab phantom set 304 move in the x-direction to intersect the X-ray beam 308. In other configurations, the bowties 320 and slab phantom set 304 move in the z-direction to intersect the X-ray beam 308. Due to physical space availability on the CT imaging system, moving bowties 320 and slab sets 304 in the z-direction may be preferred for a medium to large coverage detector 314 because physical space may not be available on the gantry 102 for the required size of the bowties 320 and slab sets 304 to fully encompass the X-ray beam 308.

As further described with respect to FIGS. 4-12, one or more of the slab phantoms of the slab phantom set 304 may be independently positioned in and/or removed from the path 312 of the X-ray beam 308. The slab phantoms of the slab phantom set 304 are shown in FIG. 3 as being positioned on either side of (e.g., not positioned in) the X-ray beam 308. One or more motors (not shown in FIG. 3) may be coupled to one or more of the slabs of the slab phantom set 304, and/or to the bowtie filter(s) 320, where the one or more motors are configured to independently move a slab (e.g., a slab phantom, a bowtie filter, etc.) into and/or out of the path 312 of the X-ray beam 308. In this way, the imaging system 200 comprises a singular holder (e.g., the housing 306) that houses the slab phantoms that are used to calibrate the imaging system 200 (e.g., the slab phantom set 304) and pre-patient collimators (e.g., the bowtie filter 320).

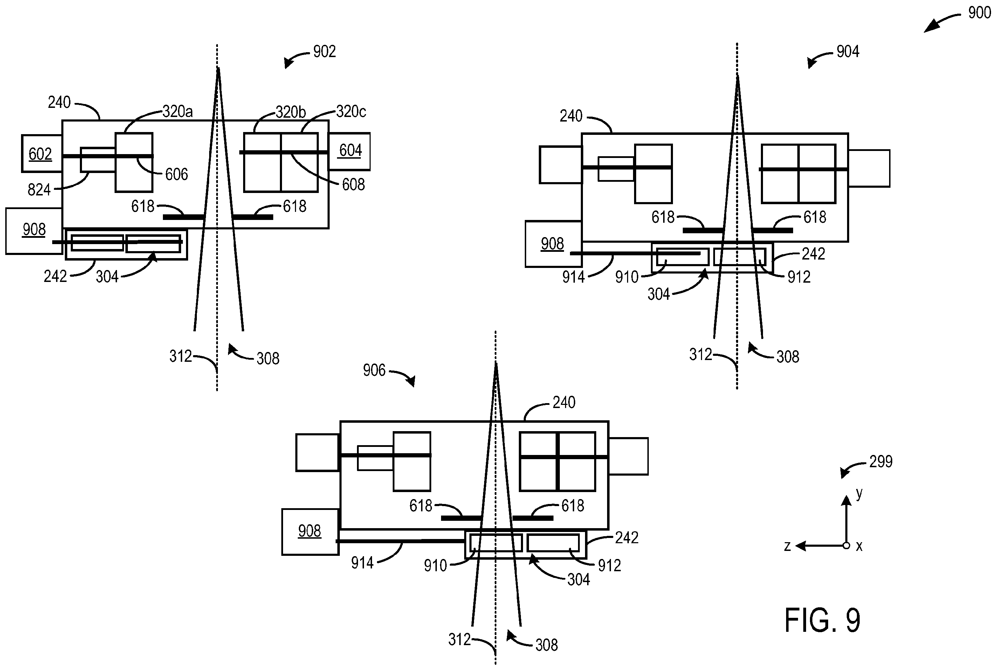

FIG. 4 shows a configuration 400 of the imaging system 200 of FIGS. 2-3, where elements of the beam-filtering device 240 (e.g., the one or more bowtie filters 320) are housed in the beam-filtering device 240, and the slab phantom set 304 is housed in the housing 242. The housing 242 is a structure that is separate from the beam-filtering device 240 in the configuration 400. The configuration 400 may include at least some of the same components as the imaging system 200 of FIGS. 2-3, which are labeled in FIG. 4 and may not be reintroduced, for brevity. The beam-filtering device 240 includes a first gap 436 on the first side 390 of the beam-filtering device 240 adjacent to the X-ray source 302. The housing 242 includes a second gap 438 on the second side 392 of the housing 242, opposite the first side 390. The housing 242 and the beam-filtering device 240 both include a third gap 440 at a respective face where the housing 242 and the beam-filtering device face each other. The first gap 436, the second gap 438, and the third gap 440 are aligned (e.g., along the y-axis) such that the X-ray beam 308 passes through the first, second, and third gaps 436, 438, 440. The housing 242 may be rigidly positioned in the path 312 of the X-ray beam 308. For example, the housing 242 may be coupled to the beam-filtering device 240, such as via a weld, bolts, clamps, and/or other rigid fasteners. In other examples, the housing 242 may be rigidly coupled to another part of rotating portion 334 of the gantry housing 322, to rigidly position the housing 242 in the path 312 of the X-ray beam 308. In further examples, as described with respect to FIGS. 9-12, the housing 242 may be selectively coupled to the beam-filtering device 240. The beam-filtering device 240 is positioned between the housing 242 and the X-ray source 302. As further described herein with respect to FIGS. 7-13, the slabs of the slab phantom set 304 may be independently and selectively positioned in the path 312 of the X-ray beam 308 during calibration operations of the imaging system 200. Thus, the X-ray beam 308 may pass through the bowtie filter(s) 320 prior to passing through one or more filters of the slab phantom set 304.

FIG. 5 shows a view 500 of the configuration 400 of FIG. 4. In the view 500 of FIG. 5, slab phantoms of the slab phantom set 304 are positioned in the path 312 of the X-ray beam 308. In the example of FIG. 5, the slab phantom set 304 includes four slabs. Each of the first slab phantom 324, the second slab phantom 326, a third slab phantom 524, and a fourth slab phantom 526 of the slab phantom set 304 may have a same width that is perpendicular to the path 312 of the X-ray beam 308 (e.g., parallel to the x-axis). The width of each slab phantom is configured to be at least equal to, or greater than, a width of the X-ray beam 308 at the position of the slab phantom. For example, a first width 530 of the first slab phantom 324 is greater than a second width 532 of the X-ray beam 308 at the position of the first slab phantom 324. In this way, the first slab phantom 324 is fully positioned in the path 312 of the X-ray beam 308 such that an entirety of the X-ray beam 308 passes through the first slab phantom 324 on the path 312 from the X-ray source 302 to the detector 314.

As described with respect to FIG. 3, one or more of the slabs of the slab phantom set 304 may be formed of the same or of different materials. Further, one or more of the slab phantoms of the slab phantom set 304 may have the same or different thicknesses, parallel to the path 312 of the X-ray beam 308 (e.g., parallel to the y-axis). Thus, different slabs may be selected or predetermined as part of a given slab phantom.

As further described herein with respect to FIGS. 7-13, the slabs of the slab phantom set 304 may be independently and selectively positioned in the path 312 of the X-ray beam 308 during calibration operations of the imaging system 200. For example, one or more of the slabs of the slab phantom set 304 may be simultaneously positioned in the path 312 of the X-ray beam 308, as shown in FIG. 5. The bowties 320 and slab sets 304 move in the z-direction to go into and out of the X-ray beam 308. This z-direction -movement configuration of bowties 320 and slab sets 304 is preferred for a detector 314 having medium to large x-direction coverage, due to physical space limitations on the rotating gantry 102.

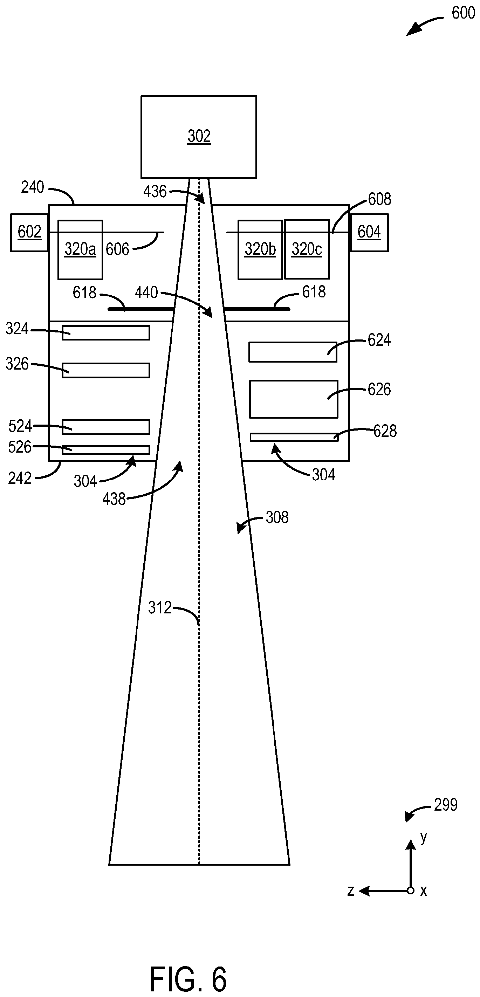

FIG. 6 shows a side view 600 of a configuration of the imaging system 200 of FIG. 2. Some elements of the imaging system 200 are excluded from the side view 600, for clarity. Some elements of the imaging system 200 that are described with respect to FIGS. 3-5 are included in the configuration of FIG. 6 and are not reintroduced, for brevity. The side view 600 of FIG. 6 shows the first slab phantom 324, the second slab phantom 326, the third slab phantom 524, and the fourth slab phantom 526 of the slab phantom set 304. The slab phantom set 304 may further include a fifth slab phantom 624, a sixth slab phantom 626, and a seventh slab phantom 628. Each of the slab phantoms of the slab phantom set 304 may have a different thickness along the path 312 of the X-ray beam 308 (e.g., parallel to the y-axis). Further, one or more of the slabs of the slab phantom set 304 may be formed of a different material configured to mimic a different human body tissue.

Each of the slabs may have a corresponding motor and drive shaft. The motor may actuate the driveshaft of a slab to move the slab to intersect with the path 312 based on a selected calibration scan protocol, as will be further described below. The configuration 400 includes one or more bowtie filters 320. As shown in FIG. 6, the beam-filtering device 240 may include three bowtie filters 320 and two apertures 618. The function of the aperture 618 is to increase or decrease the coverage of the X-ray beam 308 in the z-direction. An aperture 618 of the collimator is configured to direct the X-ray beam 308 along the path 312 from the X-ray source 302 to the detector 314. As noted, the bowtie filters 320 may be configured to alter a shape or size of the X-ray beam 308 as it passes through a subject during a diagnostic image. In other examples, the beam-filtering device 240 may include more than or less than the illustrated number of bowtie filters 320 and/or apertures 618. The bowtie filters 320 are positioned at the first side 390 of the beam-filtering device 240 and the aperture 618 is positioned at the second side 392 of the beam-filtering device 240. The aperture 618 may be movably positioned in the beam-filtering device 240, for example using a driving system such as a motor and driveshaft (not shown). Each of the bowtie filters may be coupled to a driving system comprising a motor and a driveshaft. For example, each of the bowtie filters 320 may be mounted on a driveshaft of a motor that is configured to selectively and/or independently move the bowtie filters 320 into and/or out of the path 312 of the X-ray beam 308. For example, a first motor 602 may have a first driveshaft 606 extending therefrom. A first bowtie filter 320a may be mounted on and/or coupled to the first driveshaft 606. The first motor 602 may be actuated to selectively move the first bowtie filter 320a into and/or out of the path 312 of the X-ray beam 308. A second motor 604 may have a second driveshaft 608 extending therefrom. A second bowtie filter 320b and a third bowtie filter 320c may be mounted on and/or coupled to the second driveshaft 608. The second motor 604 may be actuated to selectively move the second bowtie filter 320b and/or the third bowtie filter 320c into and/or out of the path 312 of the X-ray beam 308.

In a similar sense, the various slabs of the slab phantom set 304 may each be coupled to a driving system that includes a motor with a driveshaft configured to move corresponding slabs to intersect with the path 312 based on a selected protocol. For example, a first protocol may indicate that a first slab phantom is to be imaged for a calibration scan. The first slab phantom may comprise a first subset of slabs of the slab phantom set 304. When the first protocol is selected, respective driving systems thereof corresponding to the first subset of slabs may actuate the first subset of slabs to move into the path 312 while the other slabs of the slab phantom set 304 that are not included in the first subset are not actuated to move. In this way, the first slab phantom may be imaged for calibration of the imaging system according to the selected calibration scan protocol. When a second, different, calibration protocol is selected, a different driving system thereof may actuate a second subset of the slab phantom set 304 to move into the path 312, wherein the second subset of slabs forms a second slab phantom that corresponds to the selected second calibration protocol.

In some examples, the bowtie filters may be included in the selected calibration protocol, and thus the motors and driveshafts thereof corresponding to the bowtie filters (e.g., first and second motors 602, 604 and first and second driveshafts 606, 608) may actuate during a calibration protocol. In other examples, the bowtie filters may not be included in the selected calibration protocol and thus may not be actuated to move into the path during a calibration scan. Similarly, the motors and driveshafts corresponding to the slab phantom set 304 may not be actuated during a diagnostic imaging protocol as phantoms are intended for calibration purposes rather than diagnostic purposes.

FIGS. 7A and 7B show side views 700 of the configuration shown in FIG. 6 of the imaging system 200. Some element labels may be excluded from one or more side views 700 of FIGS. 7A and 7B for clarity. It is to be understood that each side view 700 of FIGS. 7A and 7B include the same elements. In the side views 700, various combinations of bowtie filters and/or slab phantoms are shown selectively positioned in the path 312 of the X-ray beam 308. The views of FIGS. 7A and 7B illustrate how slabs of the slab phantom set 304, as well as bowtie filters 320, may be moved independently into and/or out of the path of the X-ray beam 308, thus allowing the slabs to be used individually and/or in combination with other slab phantoms and/or bowtie filters 320. The slabs and the bowtie filters 320 are described herein as being moved into and/or out of the X-ray beam 308 by a motor with a drive shaft. Other types of drive mechanisms may be used to selectively and independently move the filters into and/or out of the X-ray beam 308 without departing from the scope of the present disclosure.

In a first view 702, the first motor 602 is actuated to extend the first driveshaft 606 and position the first bowtie filter 320a in the path 312 of the X-ray beam 308. Motors that are coupled to slabs of the slab phantom set 304 are not actuated, therefore none of the slabs of the slab phantom set 304 are positioned in the path 312 of the X-ray beam 308. The first view 702 may be an example of a configuration of the imaging system 200 during a conventional, diagnostic imaging procedure (e.g., not a calibration procedure of the imaging system). By positioning the first bowtie filter 320a in the path 312 of the X-ray beam 308, the first bowtie filter 320a may be used to alter a size, shape, or configuration of the beam during a diagnostic scan performed by the imaging system.

The imaging system 200 may include a third motor 712 with a third driveshaft 705 extending therefrom. The fifth slab phantom 624 is positioned on and/or coupled to the third driveshaft 705. In a second view 704, the third motor 712 is actuated to extend the third driveshaft 705 and position the fifth slab phantom 624 in the path 312 of the X-ray beam 308. Motors that are coupled to other slabs of the slab phantom set 304 are not actuated, therefore the first slab phantom 324, the second slab phantom 326, the third slab phantom 524, the fourth slab phantom 526, the sixth slab phantom 626, and the seventh slab phantom 628 are not positioned in the X-ray beam 308. Further, the first motor 602 and the second motor 604 are not actuated, therefore the first bowtie filter 320a, the second bowtie filter 320b, and the third bowtie filter 320c are not positioned in the X-ray beam 308. The second view 704 thus illustrates an example configuration where a single slab is positioned in the X-ray beam 308. The second view 704 may be implemented when performing a first calibration procedure of the imaging system 200.

The imaging system 200 may further include a fourth motor 714 with a fourth driveshaft 716 extending therefrom. The sixth slab phantom 626 is positioned on and/or coupled to the fourth driveshaft 716. In a third view 706, the fourth motor 714 is actuated to extend the fourth driveshaft 716 and position the sixth slab phantom 626 in the path 312 of the X-ray beam 308. Additionally, the third motor 712 is actuated to extend the third driveshaft 705 and position the fifth slab phantom 624 in the path 312 of the X-ray beam 308. Motors that are coupled to other slab phantoms of the slab phantom set 304 are not actuated, therefore the first slab phantom 324, the second slab phantom 326, the third slab phantom 524, the fourth slab phantom 526, and the seventh slab phantom 628 are not positioned in the X-ray beam 308. Further, the first motor 602 and the second motor 604 are not actuated, therefore the first bowtie filter 320a, the second bowtie filter 320b, and the third bowtie filter 320c are not positioned in the X-ray beam 308. The third view 706 thus illustrates an example configuration where multiple slabs forming a single slab phantom are positioned in the X-ray beam 308. In the example of the third view 706, the phantom slabs that are positioned in the X-ray beam 308 are housed on and extend from a same side (e.g., on a first side 790 of the X-ray beam 308) of the housing 242. The third view 706 may be implemented when performing a second calibration procedure of the imaging system 200, where the second calibration procedure is different from the first calibration procedure that uses the second view 704.

The imaging system 200 may include a fifth motor 718 with a fifth driveshaft 720 extending therefrom. The seventh slab phantom 628 is positioned on and/or coupled to the fifth driveshaft 720. In a fourth view 708, the fifth motor 718 is actuated to extend the fifth driveshaft 720 and position the seventh slab phantom 628 in the path 312 of the X-ray beam 308. Additionally, the third motor 712 is actuated to extend the third driveshaft 705 and position the fifth slab phantom 624 in the path 312 of the X-ray beam 308. Motors that are coupled to other slabs of the slab phantom set 304 are not actuated, therefore the first slab phantom 324, the second slab phantom 326, the third slab phantom 524, the fourth slab phantom 526, and the sixth slab phantom 626 are not positioned in the X-ray beam 308. Further, the first motor 602 and the second motor 604 are not actuated, therefore the first bowtie filter 320a, the second bowtie filter 320b, and the third bowtie filter 320c are not positioned in the X-ray beam 308. The fourth view 708 thus illustrates an example configuration where multiple slabs forming a slab phantom are positioned in the X-ray beam 308. In the example of the fourth view 708, the slabs that are positioned in the X-ray beam 308 are housed on and extend from opposite sides (e.g., the first side 790 of the X-ray beam 308 and a second side 792 of the X-ray beam 308, opposite the first side 790 with respect to the z-axis) of the housing 242. The fourth view 708 may be implemented when performing a third calibration procedure of the imaging system 200, where the third calibration procedure is different from the first calibration procedure that uses the second view 704 and the second calibration procedure that uses the third view 706.

By independently and selectively positioning one or more slabs of the slab phantom set 304 in the path of the X-ray beam, different combinations of slabs may be used to perform calibration procedures and/or diagnostic imaging procedures. The different combinations may form different slab phantoms with different organizations and configurations of materials, densities, and thicknesses configured to mimic different types of human body tissue. For example, a first slab phantom may comprise a selected group of slabs in a particular configuration that are configured to mimic a human torso while a second slab phantom may comprise a different selected group of slabs in another configuration configured to mimic a human brain. In this way, the different slab phantoms that are available to the system may be selected using the same set of slabs rather than needing multiple of the same slab to form different individual slab phantoms external to the imaging system.

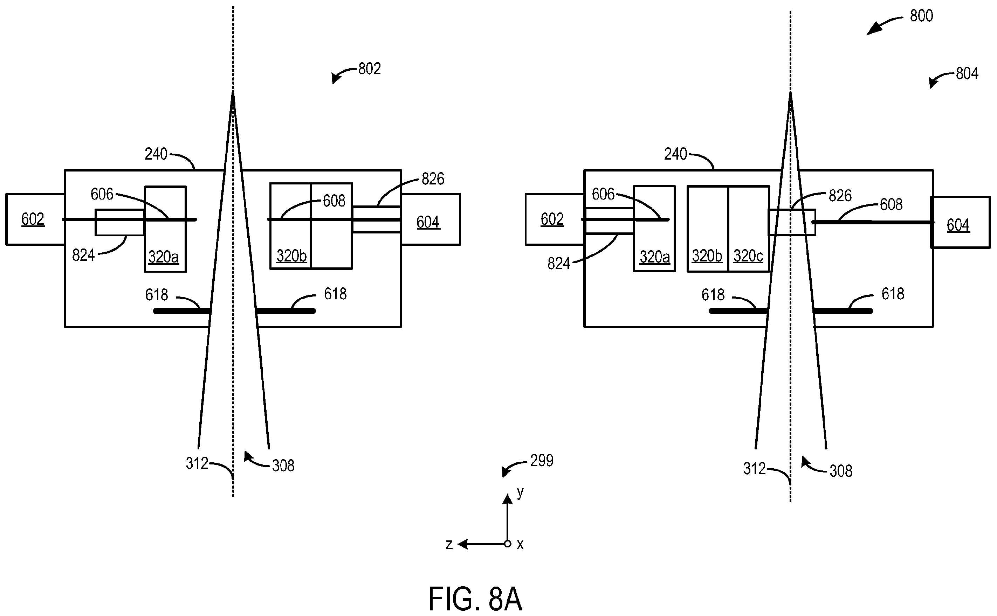

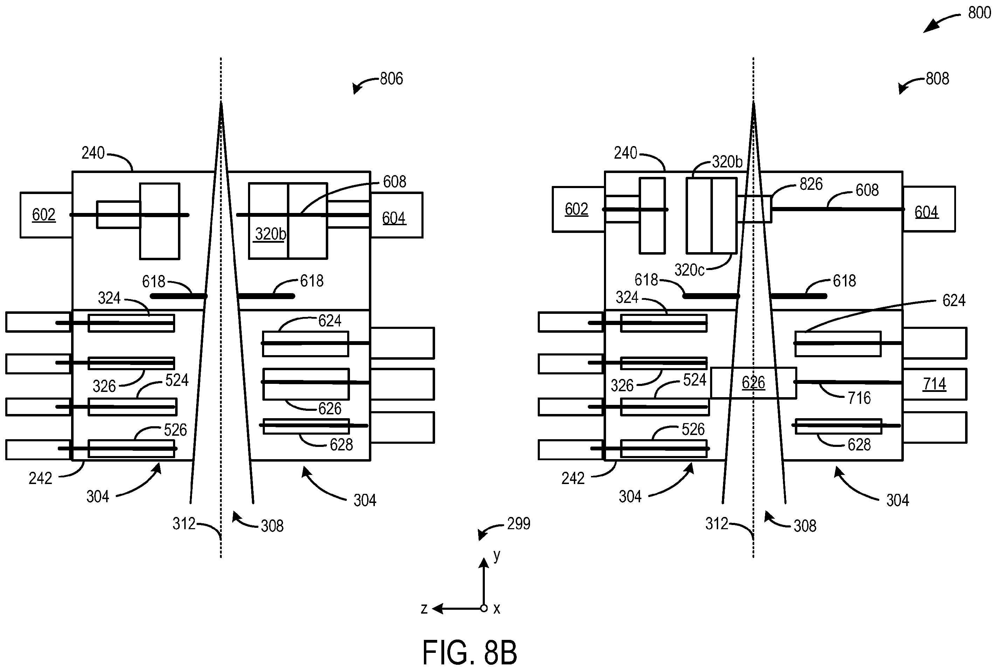

FIGS. 8A and 8B show a configuration 800 of the imaging system 200 of FIG. 2, where the beam-filtering device 240 and the housing 242 include slab phantoms of the slab phantom set 304. Some elements of the imaging system 200 that are described with respect to FIGS. 3-7 are included in the configuration of FIGS. 8A-8B and are not reintroduced, for brevity. The configuration 800 provides further illustration of how slabs of the slab phantom set 304, as well as bowtie filters 320, may be moved independently into and/or out of the path of the X-ray beam 308, thus allowing the slabs to be used individually and/or in combination (as slab phantoms) with other slabs and/or bowtie filters 320. The slabs and the bowtie filters 320 are described herein as being moved into and/or out of the X-ray beam 308 by a motor with a drive shaft. Other types of drive mechanisms may be used to selectively and independently move the filters into and/or out of the X-ray beam 308 without departing from the scope of the present disclosure.

Slabs of the slab phantom set 304 are positioned in the beam-filtering device 240, which also houses the bowtie filters 320 and the aperture 618. In addition to the first bowtie filter 320, an eighth slab 824 is coupled to the first driveshaft 606 of the first motor 602. In addition to the second bowtie filter 320b and the third bowtie filter 320c, a ninth slab 826 is coupled to the second driveshaft 608 of the second motor 604. In a first view 802, neither the first motor 602 nor the second motor 604 are actuated, therefore none of the first bowtie filter 320a, the second bowtie filter 320b, the third bowtie filter 320c, the eighth slab 824, and the ninth slab 826 are positioned in the X-ray beam 308. Each of the first motor 602 and the second motor 604 may be independently actuated to extend their driveshaft (e.g., the first driveshaft 606 and the second driveshaft 608, respectively) to independently position each of the filters and slabs mounted thereon in the X-ray beam 308. For example, in the second view 804, the second motor 604 is actuated. The second motor 604 extends the second driveshaft 608 to a first position such that the ninth slab 826 is positioned in the X-ray beam 308. The second bowtie filter 320b and the third bowtie filter 320c are not positioned in the X-ray beam 308 when the second driveshaft 608 is in the first position. In the second view 804, the second bowtie filter 320b and the third bowtie filter 320c are positioned on the second side 792 of the X-ray beam 308 (e.g., on the same side as the first bowtie filter 320a and the eighth slab 824). Extension of the second driveshaft 608 (e.g., parallel to the z-axis) may be adjusted to another position via actuation of the second motor 604 to alternatively position the third bowtie filter 320c or the second bowtie filter 320b in the X-ray beam 308. In this way, with slabs and bowtie filters aligned on the same driveshafts and within the same housing, number of components and complexity of the system may be reduced.

In a third view 806 and a fourth view 808, the housing 242 is coupled to the beam-filtering device 240, and additional slabs of the slab phantom set 304 are positioned in the housing 242, as described with respect to FIGS. 4-7B. In the third view 806, none of the motors having slabs and/or one or more bowtie filters positioned thereon are actuated, therefore no slabs or bowtie filters are positioned in the path 312 of the X-ray beam 308. In the fourth view 808, the second motor 604 is actuated to extend the second driveshaft 608 and position the ninth slab 826 in the X-ray beam 308. Additionally, the fourth motor 714 is actuated to extend the fourth driveshaft 716 and position the sixth slab phantom 626 in the path 312 of the X-ray beam 308. In this way, a slab that is positioned in the beam-filtering device 240 and a slab that is positioned in the housing 242 are extended into the path 312 of the X-ray beam 308. The slab phantom that comprises both the slab in the beam-filtering device and the slab in the phantom housing may thus be imaged in a calibration scan without having to independently assemble the slab phantom external to the imaging system.

FIG. 9 shows a configuration 900 of the imaging system 200 of FIG. 2, where the housing 242 is configured to be selectively positioned in the path 312 of the X-ray beam 308. Some elements of the imaging system 200 that are described with respect to FIGS. 3-8B are included in the configuration of FIG. 6 and are not reintroduced, for brevity. In the examples of FIG. 9, the beam-filtering device 240 includes the aperture 618, slabs, and bowtie filters. The eighth slab 824 and the first bowtie filter 320a may be positioned on the first driveshaft 606 of the first motor 602. The second bowtie filter 320b and the third bowtie filter 320c may be coupled to the second driveshaft 608 of the second motor 604.

In a first view 902, none of the motors and driveshafts are actuated and thus none of the bowtie filters or slabs are positioned to intersect with the beam. In a second view 904, a fifth motor 908 to which a tenth slab 910 and an eleventh slab 912 are coupled is actuated to extend a fifth driveshaft 914 to a first position. In the first position, the eleventh slab 912 may intersect with the X-ray beam but the tenth slab 910 may not.

In a third view 906, the fifth motor 908 is actuated to extend the fifth driveshaft 914 to a second position. In the second position, the tenth slab 910 may intersect with the X-ray beam 308 but the eleventh slab 912 may not. In this way, with different positions of driveshafts, a single driveshaft may be coupled to multiple slabs and may selectively position a selected slab to intersect with the beam based on a position that corresponds to that slab.

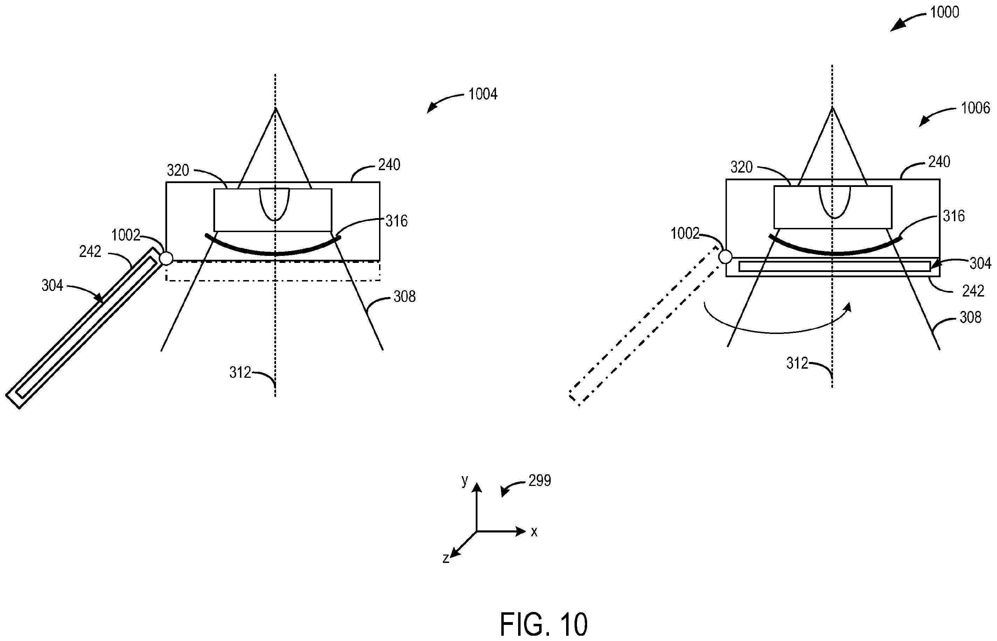

FIG. 10 shows side views of a configuration 1000 of the imaging system of FIG. 2, where the housing is configured to be selectively positioned in the path 312 of the X-ray beam 308. In the configuration 1000, the slab phantom set 304 may be integrated the housing 242. The housing 242 may be coupled to the beam-filtering device 240 via a hinge 1002. The housing 242 may be rotated from a first position 1004 to a second position 1006 by pivoting the housing 242 about the hinge 1002. In the first position 1004, the slab phantom set 304 is not positioned within the bore and thus does not intersect with the X-ray beam. However, when rotated into the second position 1006, the slab phantom set 304 may be positioned to intersect with the X-ray beam. In this example, the slabs within the set may be manually selected, as all of the set may be imaged when in the second position 1006. Pivoting the housing 242 about the hinge 1002 may additionally include removing a scan window prior to moving the housing into the path 312 of the X-ray beam 308, in some examples.

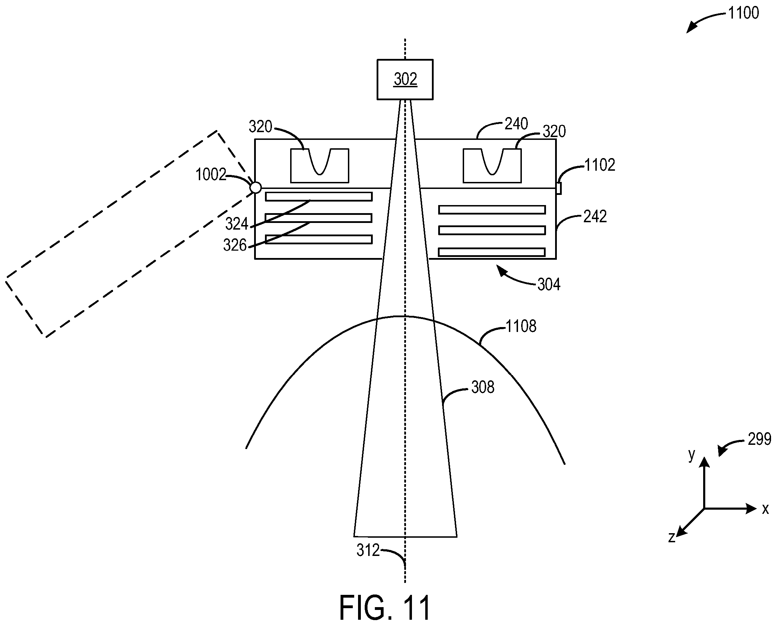

FIG. 11 shows a side view 1100 of the configuration 1000 of FIG. 10. In the side view 1100, the beam-filtering device 240 is separate from the housing 242, similar to as described above. The housing 242 may be a hinged or rotating component that is stored in the rotating gantry during diagnostic protocols. In some examples, the housing 242 may be fixedly coupled to the beam-filtering device 240 when in the configuration shown 1000 using a fastener 1102. When a calibration protocol is selected, the housing 242 may be rotated to be adjacent to the beam-filtering device 240. Then, motors and driveshafts may actuate different slabs to move into intersection with the beam as previously described. In this way, during diagnostic procedures, the slabs may be unobtrusive, reducing possibility of artifact or other interferences, and may be moved into position for calibration protocols. In some examples of the configuration 1000, the imaging system 200 includes a scan window 1108. The scan window 1108 may be removed prior to moving the housing 242 into the path 312 of the radiation beam by pivoting the housing 242 about the hinge 1002.

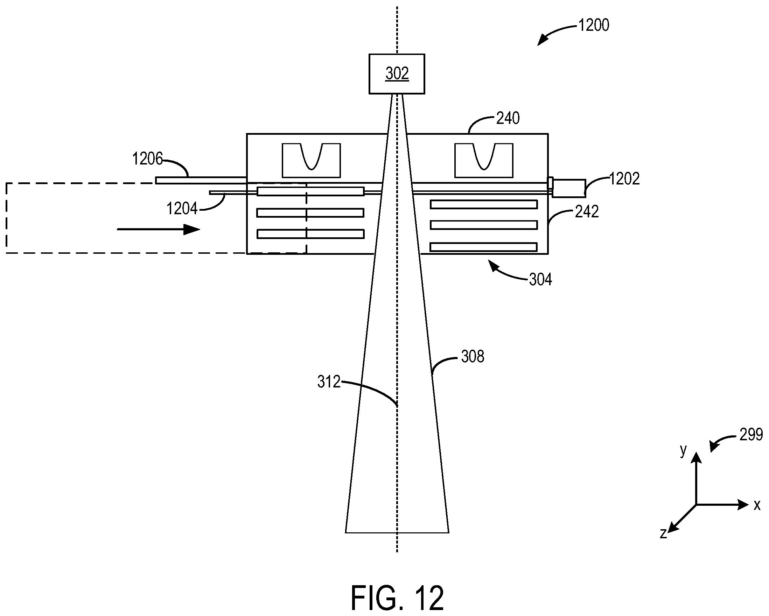

FIG. 12 shows a configuration 1200 of the imaging system of FIG. 2, where the housing 242 is configured to be selectively positioned in the path 312 of the X-ray beam 308. In the configuration 1200, a sliding mechanism may be used to move the housing 242 into position for calibration protocols. The sliding mechanism may comprise a housing motor 1202 and a housing driveshaft 1204 configured to move the housing 242 along a track 1206 that is perpendicular to the path of the radiation beam, as an example, though it should be understood that other sliding mechanisms have been envisioned.

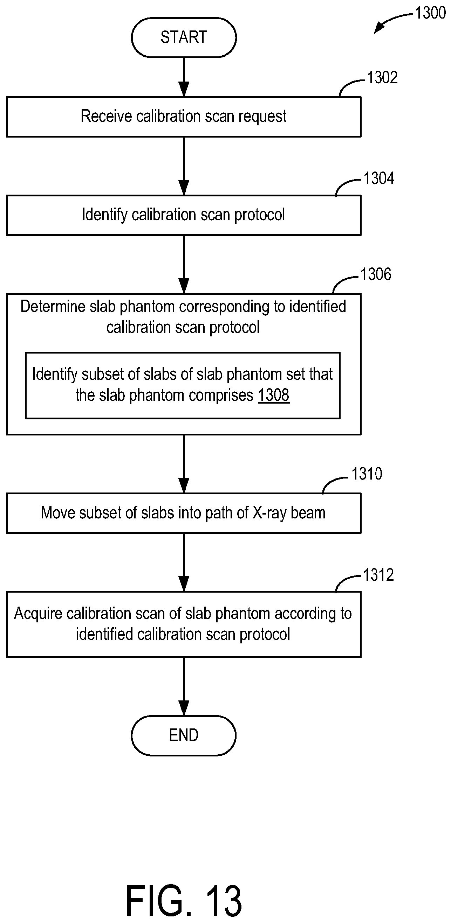

FIG. 13 shows a flowchart illustrating a method 1300 for executing a calibration scan of a slab phantom is shown. The method 1300 may be executed by one or more processors based on instructions stored in non-transitory memory. For example, the method 1300 may be carried out according to instructions stored in memory and executed by one or more processors of the computing device 216 of imaging system 200 described above.

At 1302, method 1300 includes receiving a calibration scan request. Receiving a calibration scan request may comprise receiving a user input to an operator console, in some examples. For example, a user may click an element for starting a calibration scan.

At 1304, method 1300 includes identifying a calibration scan protocol. The calibration scan protocol may be selected by the user as well. For example, the user may select a desired calibration protocol from a drop-down menu of available calibration protocols. Notably, the available calibration protocols may not include diagnostic protocols, for example calibration protocols may have different radiation amounts, beam configurations, and the like.

At 1306, method 1300 includes determining a slab phantom that corresponds to the identified calibration scan protocol. Each available calibration scan protocol may correspond to a particular slab phantom. Each slab phantom may comprise a subset of slabs from a slab phantom set (e.g., the slab phantom set 304 described above). The slab phantom set may include a variety of different slabs with different densities and thicknesses and formed of different materials. In some examples, more than one of the same slab may be included in the slab phantom set. As described above, the slab phantom set may be integrated into the imaging system. For example, the slab phantom set may be incorporated into a beam-filtering device that also houses filters such as bowtie filters which are used during diagnostic scan protocols. In another example, the slab phantom set may be incorporated into a separate phantom housing that is positioned elsewhere in the imaging system (e.g., adjacent to the beam-filtering device, within the gantry, etc.). The slab phantom set may thus comprise various combinations and sub-combinations of slabs to form different slab phantoms.

Determining the slab phantom that corresponds to the identified calibration protocol may thus include identifying a subset of slabs of the slab phantom set integrated into the imaging system, as noted at 1308. The subset of slabs may form the slab phantom that is to be imaged in the calibration scan. For example, for a first calibration scan protocol, a first subset of slabs comprising a first, a second, and a third slab may form a first slab phantom that corresponds to the first calibration scan protocol. For a second calibration scan protocol, a second subset of slabs comprising the first, the second, and a fourth slab may form a second slab phantom that corresponds to the second calibration scan protocol. When the identified calibration scan protocol is the first calibration scan protocol, the first subset of slabs may be identified. When the identified calibration scan protocol is the second calibration scan protocol, the second subset of slabs may be identified. Because the calibration scan protocol is for calibration the imaging system, filters configured for diagnostic protocols, such as bowtie filters, may not be identified.

At 1310, method 1300 includes moving the subset of slabs into a path of the X-ray beam. As described with respect to FIGS. 8-10, each slab may be coupled to a driveshaft of a motor. The motor may actuate the driveshaft to move the corresponding slab into a position that intersects with the beam. In some examples, multiple slabs may be coupled to the same driveshaft, in which case the motor may actuate the driveshaft into a particular position that corresponds to the selected slab. Thus, motors corresponding to each slab within the subset of slabs may be actuated to move each of the slabs into position intersecting the beam. Motors corresponding to slabs not in the subset of slabs may not actuated and thus the slabs not in the subset of slabs may not be moved and may remain outside the path of the beam.

For example, if the first calibration scan protocol is identified, the first, second, and third slabs may be actuated to move to intersect with the beam while the fourth slab is not moved. If the second calibration scan protocol is identified, the first, second, and fourth slabs may be actuated to move to intersect with the beam, while the third slab is not moved. In this way, the intended slabs of the slab phantom intended to be imaged in the calibration scan may be moved to intersect with the beam.

Intersection with the beam of radiation, as herein described, may include a perpendicular intersection, whereby the slab is moved linearly a direction perpendicular to the path of the radiation beam. In some examples, the driveshaft may be rotated to rotate the slab into the path of the radiation beam.

Further, as a calibration scan protocol was identified, filters meant for diagnostic protocols, such as bowtie filters, may not be actuated to move into position intersecting the beam. In some examples, a bowtie filter may be coupled to the same driveshaft as a slab of the identified subset. In such examples, the driveshaft may move into a corresponding position that includes the slab intersecting the beam and the bowtie filter not intersecting the beam.

At 1312, method 1300 includes acquiring the calibration scan of the slab phantom according to the identified calibration scan protocol. With the subset of slabs positioned in the path of the beam, the calibration scan may be acquired such that X-rays pass through the slab phantom in order to image the slab phantom. The image of the slab phantom that is outputted may be used for calibration purposes of the imaging system (e.g., may be compared to known standards to assess for drift or other common system errors).

Thus, the technical effect of the systems and methods herein provided is that slabs may be stored within the imaging system itself in an integrated manner. As such, slabs need not be stored elsewhere, thereby reducing the amount of space taken up by the slabs. Further, time spent by the operator may be reduced as the integrated phantom system may automatically and independently move individual slabs of selected slab phantoms into position without aid from the user. Automatic placement of slab phantoms as such may also increase accuracy of calibration by reducing human error in placements.

Further, as the housing in which the set of phantom slabs is positioned may be located closer to the X-ray source of the imaging system, the size (e.g., width) of the individual slabs may be reduced because the width of the X-ray beam that is to be intersected with is narrower closer to the source than at the isocenter where manually placed phantoms are typically positioned. Reducing the size of the slabs may increase space efficiency for storage within the system.

The disclosure also provides support for an imaging system, comprising: a gantry including a radiation source and a detector, a housing positioned adjacent to the radiation source within the gantry, a slab phantom set positioned in the housing, where the slab phantom set comprises at least one slab phantom, and wherein the slab phantom set is configured such that the at least one slab phantom of the slab phantom set is independently movable to enable one or more of the at least one slab phantom of the slab phantom set to be selectively positioned in a path of a radiation beam between the radiation source and the detector. In a first example of the system, the housing includes a collimator configured to align and/or narrow the radiation beam, and where the housing comprises one or more bowtie filters and an aperture. In a second example of the system, optionally including the first example, the one or more bowtie filters are positioned in the housing at a first end, the slab phantom set is positioned at a second end of the housing, and the aperture is positioned between the one or more bowtie filters and the slab phantom set. In a third example of the system, optionally including one or both of the first and second examples, the one or more bowtie filters are positioned in the housing at a first end, the slab phantom set is positioned in horizontal alignment with the one or more bowtie filters, and the aperture is positioned at a second end of the housing in vertical alignment with the one or more bowtie filters. In a fourth example of the system, optionally including one or more or each of the first through third examples, the at least one slab phantom includes a first slab phantom and a second slab phantom, further including at least one motor, wherein at least one motor is configured to selectively position one or more of a selected bowtie filter of the one or more bowtie filters, the first slab phantom, and the second slab phantom in the path of the radiation beam. In a fifth example of the system, optionally including one or more or each of the first through fourth examples, the system further comprises: a motor coupled at least one of the one or more bowtie filters, wherein the motor is configured to move a selected bowtie filter of the one or more bowtie filters to selectively position the selected bowtie filter of the one or more bowtie filters in the path of the radiation beam. In a sixth example of the system, optionally including one or more or each of the first through fifth examples, the at least one slab phantom includes a first slab phantom and a second slab phantom, further including a first motor coupled to the first slab phantom, wherein the first motor is configured to selectively position the first slab phantom in the path of the radiation beam between the radiation source and the detector, and where a first width of the first slab phantom is approximately equal to a width of the radiation beam at which the first slab phantom is selectively positioned. In a seventh example of the system, optionally including one or more or each of the first through sixth examples, the system further comprises: a second motor coupled to the second slab phantom, wherein the second motor is configured to move the second slab phantom to selectively position the second slab phantom in the path of the radiation beam between the radiation source and the detector, and where a second width of the second slab phantom is approximately equal to the width of the radiation beam at which the second slab phantom is selectively positioned. In an eighth example of the system, optionally including one or more or each of the first through seventh examples, the housing is rigidly coupled to a beam-filtering device and where the beam-filtering device is positioned between the housing and the radiation source. In a ninth example of the system, optionally including one or more or each of the first through eighth examples, the housing is configured to be selectively positioned in the path of the radiation beam, and is configured to move into and out of the path of the radiation beam by pivoting about a hinge. In a tenth example of the system, optionally including one or more or each of the first through ninth examples, the housing is configured to be selectively positioned in the path of the radiation beam, and is configured to move into and out of the path of the radiation beam by moving linearly along a track. In an eleventh example of the system, optionally including one or more or each of the first through tenth examples, the at least one slab phantom includes a first slab phantom, a second slab phantom, and a third slab phantom of the slab phantom set, wherein the third slab phantom is independently movable relative to the first slab phantom and the second slab phantom to selectively position the third slab phantom in the path of the radiation beam. In a twelfth example of the system, optionally including one or more or each of the first through eleventh examples, the at least one slab phantom includes a first slab phantom and a second slab phantom, wherein the first slab phantom is formed of a first material, and the second slab phantom is formed of a second material that is different from the first material. In a thirteenth example of the system, optionally including one or more or each of the first through twelfth examples, the at least one slab phantom includes a first slab phantom and a second slab phantom, wherein the first slab phantom has a first thickness, and the second slab phantom has a second thickness, different from the first thickness.

The disclosure also provides support for a method for calibrating an imaging system, comprising: identifying a calibration scan protocol requested, determining a slab phantom corresponding to the identified calibration scan protocol, identifying a subset of slabs of a slab phantom set incorporated into a housing of the imaging system that form the slab phantom corresponding to the identified calibration scan protocol, moving the subset of slabs into a path of a radiation beam, and acquiring a calibration scan of the slab phantom. In a first example of the method, the subset of slabs comprises a first slab phantom, and wherein moving the first slab phantom comprises actuating a first motor coupled to the first slab phantom via a first shaft, the first shaft operative to move the first slab phantom in a direction perpendicular to the path of the radiation beam. In a second example of the method, optionally including the first example, the method further comprises: moving the housing into the path of the radiation beam by pivoting the housing about a hinge and/or linearly translating the housing along a track that is perpendicular to the path of the radiation beam, where the slab phantom set is positioned in the housing. In a third example of the method, optionally including one or both of the first and second examples, the method further comprises: removing a scan window prior to moving the housing into the path of the radiation beam by pivoting the housing about the hinge.

The disclosure also provides support for an imaging system, comprising: a gantry including a bore configured to receive an imaging subject, a radiation source positioned in the gantry and configured to emit a radiation beam, a detector positioned in the gantry, opposite the radiation source, a housing positioned in the gantry adjacent the radiation source, wherein a first slab phantom and a second slab phantom are positioned in the housing, a first driving system configured to move the first slab phantom into and out of a path of the radiation beam, a second driving system configured to move the second slab phantom into and out of the radiation beam, and a computing device with instructions stored in a non-transitory memory that, when executed by a processor, cause the processor to: identify a calibration scan protocol, and actuate one or more of the first driving system and the second driving system to move the first slab phantom and/or the second slab phantom, respectively, into the path of the radiation beam based on the identified calibration scan protocol, and execute a calibration procedure. In a first example of the system, the housing includes a at least one bowtie filter and an aperture, where the aperture is positioned between the at least one bowtie filter and the first slab phantom and the second slab phantom.

FIGS. 1-12 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. An imaging system (200), comprising:
a gantry (322) including a radiation source (302) and a detector (314);
a housing (306) positioned adjacent to the radiation source within the gantry;
a slab phantom set (304) positioned in the housing, where the slab phantom set comprises at least one slab phantom (324, 326), and wherein the slab phantom set is configured such that the at least one slab phantom of the slab phantom set is independently movable to enable one or more of the at least one slab phantom of the slab phantom set to be selectively positioned in a path (312) of a radiation beam (308) between the radiation source and the detector.

2. The imaging system of claim 1, wherein the housing includes a collimator configured to align and/or narrow the radiation beam, and where the housing comprises one or more bowtie filters (320) and an aperture (618).

3. The imaging system of claim 2, wherein the one or more bowtie filters are positioned in the housing at a first end (390), the slab phantom set is positioned at a second end (392) of the housing, and the aperture is positioned between the one or more bowtie filters and the slab phantom set.

4. The imaging system of claim 2, wherein the one or more bowtie filters are positioned in the housing at a first end (390), the slab phantom set is positioned in horizontal alignment with the one or more bowtie filters, and the aperture is positioned at a second end (392) of the housing in vertical alignment with the one or more bowtie filters.

5. The imaging system of claim 2, wherein the at least one slab phantom includes a first slab phantom (324) and a second slab phantom (326), further including at least one motor (602, 604), wherein at least one motor is configured to selectively position one or more of a selected bowtie filter of the one or more bowtie filters, the first slab phantom, and the second slab phantom in the path of the radiation beam.

6. The imaging system of claim 2, further comprising a motor (602) coupled at least one of the one or more bowtie filters, wherein the motor is configured to move a selected bowtie filter of the one or more bowtie filters to selectively position the selected bowtie filter of the one or more bowtie filters in the path of the radiation beam.

7. The imaging system of claim 1, wherein the at least one slab phantom includes a first slab phantom (705) and a second slab phantom (716), further including a first motor coupled to the first slab phantom, wherein the first motor is configured to selectively position the first slab phantom in the path of the radiation beam between the radiation source and the detector, and where a first width (530) of the first slab phantom is approximately equal to a width (532) of the radiation beam at which the first slab phantom is selectively positioned.

8. The imaging system of claim 7, further comprising a second motor (714) coupled to the second slab phantom (716), wherein the second motor is configured to move the second slab phantom to selectively position the second slab phantom in the path of the radiation beam between the radiation source and the detector, and where a second width of the second slab phantom is approximately equal to the width of the radiation beam at which the second slab phantom is selectively positioned.

9. The imaging system of claim 1, wherein the housing (242) is rigidly coupled to a beam-filtering device (240) and where the beam-filtering device is positioned between the housing and the radiation source.

10. The imaging system of claim 1, wherein the housing is configured to be selectively positioned in the path of the radiation beam, and is configured to move into and out of the path of the radiation beam by pivoting about a hinge (1002).

11. The imaging system of claim 1, wherein the housing is configured to be selectively positioned in the path of the radiation beam, and is configured to move into and out of the path of the radiation beam by moving linearly along a track (1206).

12. The imaging system of claim 1, wherein the at least one slab phantom includes a first slab phantom (324), a second slab phantom (326), and a third slab phantom (524) of the slab phantom set, wherein the third slab phantom is independently movable relative to the first slab phantom and the second slab phantom to selectively position the third slab phantom in the path of the radiation beam.

13. The imaging system of claim 1, wherein the at least one slab phantom includes a first slab phantom (324) and a second slab phantom (326), wherein the first slab phantom is formed of a first material, and the second slab phantom is formed of a second material that is different from the first material.

14. The imaging system of claim 1, wherein the at least one slab phantom includes a first slab phantom and a second slab phantom, wherein the first slab phantom has a first thickness, and the second slab phantom has a second thickness, different from the first thickness.

15. A method (1300) for calibrating an imaging system (200), comprising:
identifying (1304) a calibration scan protocol requested;
determining (1306) a slab phantom corresponding to the identified calibration scan protocol;
identifying (1308) a subset of slabs of a slab phantom set incorporated into a housing of the imaging system that form the slab phantom corresponding to the identified calibration scan protocol;
moving (1310) the subset of slabs into a path of a radiation beam; and
acquiring (1312) a calibration scan of the slab phantom.
